# EUROPEAN PATENT APPLICATION

(11) **EP 1 767 649 A1**
(43) Date of publication of application: **28.03.2007**
(21) Application number: 05748512.0
(22) Date of filing: 02.06.2005
(51) Int. Cl.: C12Q 1/48, G01N 33/15, G01N 33/50, C12N 15/09

(54) **ASSAY OF UBIQUITINIZATION OF SYNOVIOLIN AND USE THEREOF IN SCREENING**

(30) Priority: 02.06.2004 JP 2004165063
(71) Applicant: Locomogene, Inc., Tokyo 105-0001 (JP)
(72) Inventor: NAKAJIMA, Toshihiro Kohoku Garden Hills, kanagawa 2240001 (JP); TORIYAMA, Sayumi, awasaki-shi, Kanagawa 2160014 (JP); YAMADERA, Tadayuki Okid Garden, Kanagawa 2160015 (JP); OHTA, Tomohiko, 1550032 (JP); YAMASAKI, Satoshi Ishikawazaka Manshon, Kanagawa 2250003 (JP); YAGISHITA, Naoko Kosumo Konandai, Kanagawa 2340053 (JP); ZHANG, Lei Toranomon Pastoral Main, Tokyo 1050001 (JP); IKEDA, Rie Haitsu Maria, Kanagawa 2160015 (JP); SASAKI, Ken Fine Higashiyurigaoka, Kawasaki-shi kanagawa 2150012 (JP); AMANO, Tetsuya Kaede, Kanagawa 2140037 (JP)
(74) Representative: Woods, Geoffrey Corlett
(86) International application number: PCT/JP2005/010540
(87) International publication number: WO 2005/118841

(57) **Abstract**

The present invention relates to an assay method for determining a auto-ubiquitination activity of synoviolin, comprising reacting synoviolin and ubiquitin in a reaction system containing them and determining an amount of ubiquitin binding to synoviolin, to a method of screening a substance capable of regulating such an activity, and to a kit for auto-ubiquitination assay of synoviolin.

SEQUENCE LISTING

<110> LocomoGene Inc.

<120> ASSAY OF UBIQUITINIZATION OF SYNOVIOLIN AND USE THEREOF IN
SCREENING

<130> G06-0077EP

<150> JP2004-165063
<151> 2004-06-02

<160> 4

<170> PatentIn version 3.3

<210> 1
<211> 3374
<212> DNA
<213> Homo sapiens

<220>
<221> CDS
<222> (403)..(2256)

<400> 1
gccctttctt atgagcatgc ctgtgttggg ttgacagtga gggtaataat gacttgttgg 60
ttgattgtag atatagggct ctcccttgca aggtaattag gctccttaaa ttacctgtaa 120
gattttcttg ccacagcatc cattctggtt aggctggtga tcttctgagt agtgatagat 180
tggttggtgg tgaggtttac aggtgttccc ttctcttact cctggtgttg gctacaatca 240
ggtggcgtct agagcagcat gggacaggtg ggtaagggga gtcttctcat tatgcagaag 300
tgatcaactt aaatctctgt cagatctacc tttatgtagc ccggcagtcg cgcggattga 360
gcgggctcgc ggcgctgggt tcctggtctc cgggccaggg ca atg ttc cgc acg 414
gca gtg atg atg gcg gcc agc ctg gcg ctg acc ggg gct gtg gtg gct 462
cac gcc tac tac ctc aaa cac cag ttc tac ccc act gtg gtg tac ctg 510
acc aag tcc agc ccc agc atg gca gtc ctg tac atc cag gcc ttt gtc 558
ctt gtc ttc ctt ctg ggc aag gtg atg ggc aag gtg ttc ttt ggg caa 606
ctg agg gca gca gag atg gag cac ctt ctg gaa cgt tcc tgg tac gcc 654
gtc aca gag act tgt ctg gcc ttc acc gtt ttt cgg gat gac ttc agc 702
ccc cgc ttt gtt gca ctc ttc act ctt ctt ctc ttc ctc aaa tgt ttc 750
cac tgg ctg gct gag gac cgt gtg gac ttt atg gaa cgc agc ccc aac 798
atc tcc tgg ctc ttt cac tgc cgc att gtc tct ctt atg ttc ctc ctg 846
ggc atc ctg gac ttc ctc ttc gtc agc cac gcc tat cac agc atc ctg 894
acc cgt ggg gcc tct gtg cag ctg gtg ttt ggc ttt gag tat gcc atc 942
ctg atg acg atg gtg ctc acc atc ttc atc aag tat gtg ctg cac tcc 990
gtg gac ctc cag agt gag aac ccc tgg gac aac aag gct gtg tac atg 1038
ctc tac aca gag ctg ttt aca ggc ttc atc aag gtt ctg ctg tac atg 1086
gcc ttc atg acc atc atg atc aag gtg cac acc ttc cca ctc ttt gcc 1134
atc cgg ccc atg tac ctg gcc atg aga cag ttc aag aaa gct gtg aca 1182
gat gcc atc atg tct cgc cga gcc atc cgc aac atg aac acc ctg tat 1230
cca gat gcc acc cca gag gag ctc cag gca atg gac aat gtc tgc atc 1278
atc tgc cga gaa gag atg gtg act ggt gcc aag aga ctg ccc tgc aac 1326
cac att ttc cat acc agc tgc ctg cgc tcc tgg ttc cag cgg cag cag 1374
acc tgc ccc acc tgc cgt atg gat gtc ctt cgt gca tcg ctg cca gcg 1422
cag tca cca cca ccc ccg gag cct gcg gat cag ggg cca ccc cct gcc 1470
ccc cac ccc cca cca ctc ttg cct cag ccc ccc aac ttc ccc cag ggc 1518
ctc ctg cct cct ttt cct cca ggc atg ttc cca ctg tgg ccc ccc atg 1566
ggc ccc ttt cca cct gtc ccg cct ccc ccc agc tca gga gag gct gtg 1614
gct cct cca tcc acc agt gca gca gcc ctt tct cgg ccc agt gga gca 1662
gct aca acc aca gct gct ggc acc agt gct act gct gct tct gcc aca 1710
gca tct ggc cca ggc tct ggc tct gcc cca gag gct ggc cct gcc cct 1758
ggt ttc ccc ttc cct cct ccc tgg atg ggt atg ccc ctg cct cca ccc 1806
ttt gcc ttc ccc cca atg cct gtg ccc cct gcg ggc ttt gct ggg ctg 1854
acc cca gag gag cta cga gct ctg gag ggc cat gag cgg cag cac ctg 1902
gag gcc cgg ctg cag agc ctg cgt aac atc cac aca ctg ctg gac gcc 1950
gcc atg ctg cag atc aac cag tac ctc acc gtg ctg gcc tcc ttg ggg 1998
ccc ccc cgg cct gcc act tca gtc aac tcc act gag ggg act gcc act 2046
aca gtt gtt gct gct gcc tcc tcc acc agc atc cct agc tca gag gcc 2094
acg acc cca acc cca gga gcc tcc cca cca gcc cct gaa atg gaa agg 2142
cct cca gct cct gag tca gtg ggc aca gag gag atg cct gag gat gga 2190
gag ccc gat gca gca gag ctc cgc cgg cgc cgc ctg cag aag ctg gag 2238
tct cct gtt gcc cac tga cactgcccca gcccagcccc agcctctgct 2286
cttttgagca gccctcgctg gaacatgtcc tgccaccaag tgccagctcc ctctctgtct    2346
gcaccaggga gtagtacccc cagctctgag aaagaggcgg catcccctag gccaagtgga    2406
aagaggctgg ggttcccatt tgactccagt cccaggcagc catggggatc tcgggtcagt    2466
tccagccttc ctctccaact cttcagccct gtgttctgct ggggccatga aggcagaagg    2526
tttagcctct gagaagccct cttcttcccc cacccctttc caggagaagg ggctgcccct    2586
ccaagcccta cttgtatgtg cggagtcaca ctgcagtgcc gaacagtatt agctcccgtt    2646
cccaagtgtg gactccagag gggctggagg caagctatga acttgctcgc tggcccaccc    2706
ctaagactgg tacccatttc cttttcttac cctgatctcc ccagaagcct cttgtggtgg    2766
tggctgtgcc ccctatgccc tgtggcattt ctgcgtctta ctggcaacca cacaactcag    2826
ggaaaggaat gcctgggagt gggggtgcag gcgggcagca ctgagggacc ctgccccgcc    2886
cctcccccca ggcccctttc ccctgcagct tctcaagtga gactgacctg tctcacccag    2946
cagccactgc ccagccgcac tccaggcaag ggccagtgcg cctgctcctg accactgcaa    3006
tcccagcgcc caaggaaggc cacttctcaa ctggcagaac ttctgaagtt tagaattgga    3066
attacttcct tactagtgtc ttttggctta aattttgtct tttgaagttg aatgcttaat    3126
cccgggaaag aggaacagga gtgccagact cctggtcttt ccagtttaga aaaggctctg    3186
tgccaaggag ggaccacagg agctgggacc tgcctgcccc tgtcctttcc ccttggtttt    3246
gtgttacaag agttgttgga gacagtttca gatgattatt taatttgtaa atattgtaca    3306
aattttaata gcttaaattg tatatacagc caaataaaaa cttgcattaa caaaaaaaaa    3366
aaaaaaaa                                                              3374

<210>  2
<211>  617
<212>  PRT
<213>  Homo sapiens

<400>  2

<210>  3
<211>  228
<212>  DNA
<213>  Homo sapiens

<220>
<221>  CDS
<222>  (1)..(228)

<400>  3
atg cag atc ttt gtg aag acc ctc act ggc aaa acc atc acc ctt gag        48
gtc gag ccc agt gac acc att gag aat gtc aaa gcc aaa att caa gac        96
aag gag ggt atc cca cct gac cag cag cgt ctg ata ttt gcc ggc aaa       144
cag ctg gag gat ggc cgc act ctc tca gac tac aac atc cag aaa gag       192
tcc acc ctg cac ctg gtg ttg cgc ctg cga ggt ggc                       228

<210>  4
<211>  76
<212>  PRT
<213>  Homo sapiens

<400>  4

## Description

### FIELD OF THE INVENTION

The present invention relates to ubiquitination of synoviolin. More specifically, the present invention relates to a highly sensitive method for auto-ubiquitination assay of synoviolin, a rheumatoid-arthritis-associated protein, and to a high-throughput method for screening a substance that has an influence on auto-ubiquitination.

### BACKGROUND OF THE INVENTION

Rheumatoid arthritis (hereinafter, simply referred to as "RA") is a chronic systemic inflammatory disorder associated with an abnormal proliferation of a synovial tissue of a joint. Synovial cells are fibroblast-like cells that form 1 to 6 epithelial-like layers in a synovial membrane of a joint, which are thought to supply proteoglycan and hyaluronic acid to the synovial fluid. In a joint of an RA patient, symptoms such as proliferation of the synovial tissue, a multilayer structure resulting from such proliferation, infiltration of the synovial cells to other tissues or the like can be observed.

In the course of studying the underlying mechanism of onset and development of RA at the molecular level, a novel gene was found to be strongly expressed in RA patients' synovial tissue, and a protein coded by that gene was named synoviolin after the tissue expressing the gene, namely synovial cell (WO02/052007). This gene and the expression product thereof, i.e., synoviolin, are closely related to abnormal proliferation of the synovial tissue, which is a major cause of RA disorder. Synoviolin is also reported of its involvement in signal transduction. Since synoviolin has a cell-proliferating activity, information inside and outside a cell appears to be transduced by phosphorylation of a protein via synoviolin. Synoviolin is also involved in protein ubiquitination, implying that it also controls the functions of the protein.

### SUMMARY OF THE INVENTION

As described above, there has been a demand for developing a method for auto-ubiquitination assay of synoviolin protein and a method for screening a compound having an activity of regulating the ubiquitination activity.

The present inventors have gone through keen study on signal transduction by synoviolin, as a result of which we found for the first time that synoviolin has a ubiquitin ligase activity that causes auto-ubiquitination. Based on this finding, we accomplished the present invention relating to a highly sensitive method for assaying auto-ubiquitination activity of synoviolin and to a high-throughput screening method for searching a compound that regulates the auto-ubiquitination by using such an assay method for enzyme activity.

Thus, the present invention is as follows:
(1) An assay method for determining a ubiquitination activity of synoviolin, comprising reacting synoviolin and ubiquitin in a reaction system containing them, and determining an amount of ubiquitin binding to synoviolin in the resulting reaction product.
   The above-described reaction may be an auto-ubiquitination reaction in a system comprising synoviolin or an active derivative thereof, ubiquitin, a ubiquitin-activating enzyme, a ubiquitin-conjugating enzyme and a low molecular weight reactive factor required for ubiquitination. In the above-described determination step, an amount of ubiquitin binding to synoviolin may be determined.
   An example of the above-mentioned active derivative of synoviolin includes a fusion protein of synoviolin and a tag protein.
   Furthermore, the above-described determination step may be carried out by reacting biotinylated ubiquitin binding to synoviolin with avidin to quantify the amount of ubiquitin binding to synoviolin.
(2) A method for screening a substance having an activity of regulating ubiquitination of synoviolin, comprising the steps of
   (i) contacting ubiquitin with synoviolin or an active derivative thereof in the presence or in the absence of a candidate substance;
   (ii) determining the ubiquitination activity of the synoviolin or the active derivative thereof; and
   (iii) comparing the results obtained from (i) and (ii) above to that from a control to detect an activity of the candidate substance to regulate ubiquitination activity of synoviolin.
(3) A kit for ubiquitination assay of synoviolin for determining ubiquitination of synoviolin, comprising at least:
   (i) synoviolin or an active derivative thereof and ubiquitin; and
   (ii) reagents to be used in the step of determining the amount of ubiquitin binding to synoviolin.

The above kit may further comprise a ubiquitin-activating enzyme, a ubiquitin-conjugating enzyme, a low molecular weight reactive factor required for ubiquitination and buffers.

### BRIEF DESCRIPTION OF THE DRAWINGS

Figure 1 is a view showing biotinylation of GST-HA-Ub.
Figure 2 is a view showing the results of *in vitro* auto-ubiquitination of synoviolin using biotinylated GST-HA-Ub by western blotting.
Figure 3 is a view showing the results of *in vitro* auto-ubiquitination of synoviolin using biotinylated GST-HA-Ub by ELISA.
Figure 4 is a view showing the results of *in vitro* auto-ubiquitination of synoviolin using Pk-His-HA-Ub K48R by western blotting.
Figure 5 is a view showing the results of *in vitro* auto-ubiquitination of synoviolin using Pk-His-HA-Ub K48R by ELISA.
Figure 6 is a view showing a small-scale *in vivo* biotinylated BioEASE-Ub purification.
Figure 7 is a view showing *in vivo* biotinylated BioEASE-Ub purifications.
Figure 8 is a view showing *in vitro* auto-ubiquitination of synoviolin using different biotinylated ubiquitins.
Figure 9 is a view showing the effect of synoviolin concentration in *in vitro* auto-ubiquitination reaction detecting system using ELISA method.
Figure 10 is a view showing the effect of ubiquitin concentration in *in vitro* auto-ubiquitination reaction detecting system using ELISA method.
Figure 11 is a view showing detection of *in vitro* auto-ubiquitination activity of MBP-Syno ΔTM-His using ELISA method.
Figure 12 is a view showing the effect of antibody concentration upon detecting *in vitro* auto-ubiquitination reaction using ELISA.

### BEST MODES FOR CARRING OUT THE INVENTION

Hereinafter, the present invention will be described in more detail.

The present invention was accomplished based on the finding that ubiquitin binding to ubiquitinated (e.g., auto-ubiquitinated) synoviolin can be determined highly sensitively and easily by ELISA method or the like.

Ubiquitination is a process in which enzymes such as ubiquitin-activating enzyme (E1), ubiquitin-conjugating enzyme (E2) and ubiquitin ligase (E3) cooperate to successively bind ubiquitin to a substrate protein. The Physiological significance of ubiquitination has conventionally been recognized as a tag modification for transportation to the proteasome system, i.e., a protein degradation mechanism. Subsequent studies have further expanded the significance of ubiquitination, which is currently characterized as a reversible protein modification system for controlling protein functions.

Herein, the term "auto-ubiquitination" or "auto-ubiquitination" means that synoviolin having a ubiquitin ligase activity becomes the substrate protein for ubiquitination by itself and binds ubiquitin without depending on other ubiquitin ligase. Auto-ubiquitination of synoviolin became apparent when the present inventors found that synoviolin has a ubiquitin ligase activity. This auto-ubiquitination is not only observed in a full-length molecule of endogenous synoviolin but also found to occur in an intracytoplasmic part of synoviolin fused to a tag protein. Based on these findings, the present inventors have gone through keen study, thereby accomplishing the present invention.

The method of assaying a ubiquitination activity according to the invention comprises reacting synoviolin and ubiquitin in a reaction system including them, and determining the amount of ubiquitin binding to synoviolin in the resulting reaction product. The present invention further comprises the steps of
(i) allowing auto-ubiquitination of synoviolin in a system comprising synoviolin or an active derivative thereof, ubiquitin, a ubiquitin-activating enzyme, a ubiquitin-conjugating enzyme and a low molecular weight reactive factor required for ubiquitination; and
(ii) determining an amount of ubiquitin binding or not binding to synoviolin in the reaction solution obtained by the above reaction.

An amount of ubiquitin binding or not binding to synoviolin may be determined using, for example, labeled ubiquitin or an anti-ubiquitin antibody to detect ubiquitination of a substrate protein and to assess the degree of ubiquitination. Since this method is associated with direct and uncomplicated operation, the method is suitable to be used for screening a low molecular weight substance that influences auto-ubiquitination as described later below.

The phrase "synoviolin or an active derivative thereof' comprises, other than synoviolin, various derivatives having ubiquitinating enzyme activities. These include salt forms of synoviolin, modified protein molecules and fusion proteins (e.g., fusion protein of synoviolin and His-tag protein). Synoviolin used in the assay of ubiquitination is prepared as follows.

### 1. Preparation of synoviolin from biomaterial

Synoviolin may be obtained from a synovial tissue from an RA patient. Since synovial cells can be cultured *in vitro,* synoviolin may be collected from this culture. Specifically, synovial cells are isolated from a synovial tissue or the like surgically excised from an RA patient by synovectomy. The isolated cells are cultured so as to collect synovial cells as adherent cells (J. Clin. Invest. 92:186-193, 1993). From the collected cells, synoviolin is extracted and purified by combining known protein purification techniques.

### 2. Preparation of synoviolin by genetic engineering process

In order to conveniently and efficiently obtain synoviolin, it is preferable to employ the genetic engineering process described below. Furthermore, it is more preferable to prepare a recombinant of synoviolin by using a host such as bacteria because it makes addition of various tag proteins as labels to synoviolin and preparation of synoviolin mutant easy.

### (1) Synoviolin polypeptide

As described above, other than obtaining synoviolin from a biomaterial, a gene coding for synoviolin may be integrated into an appropriate expression system to obtain synoviolin as an expression product by genetic recombination.

The source of synoviolin used with the present invention is not particularly limited and may include human, mouse and rat. Synoviolin used may be, for example, a polypeptide having an amino acid sequence represented by SEQ ID NO: 2. A nucleotide sequence of DNA coding for the polypeptide represented by SEQ ID NO:2 is represented by SEQ ID NO:1. The polypeptide used with the invention may be a variant as long as it has an activity as synoviolin, and may be a polypeptide having homology with the above synoviolin amino acid sequence. Examples of such polypeptides include amino acid sequences having about 85% or more, preferably about 90% or more and more preferably about 95% or more homology with the amino acid sequence of the above synoviolin polypeptide.

Herein, to have an "activity as synoviolin" means to have a ubiquitin ligase activity.

In addition, an amino acid sequence variant of a polypeptide represented by SEQ ID NO: 2, with one or more (e.g., one or a few) amino acids mutated (e.g., deleted, substituted or added), having an activity as synoviolin may also be used with the present invention. For example, a mutant synoviolin polypeptide having the same activity as synoviolin described above and having the following amino acid sequence may also be used: (i) an amino acid sequence variants with one or more (preferably one or a few (e.g., 1 to 10, more preferably 1 to 5)) amino acids deleted in the amino acid sequence represented by SEQ ID NO:2; (ii) an amino acid sequence variants with one or more (preferably one or a few (e.g., 1 to 10, more preferably 1 to 5)) amino acids in the amino acid sequence represented by SEQ ID NO:2 substituted with other amino acids; (iii) an amino acid sequence variants with one or more (preferably one or a few (e.g., 1 to 10, more preferably 1 to 5)) amino acids added to the amino acid sequence represented by SEQ ID N0:2; or (iv) an amino acid sequence having a combination of (i) to (iii) above.

A polynucleotide coding for an amino acid sequence having one or more amino acids deleted, inserted or added in the amino acid sequence represented by SEQ ID NO: 2 may be prepared according to a method such as site-directed mutagenesis described, for example, in "Molecular Cloning, A Laboratory Manual 2nd ed." (Cold Spring Harbor Press (1989)) and "Current Protocols in Molecular Biology" (John Wiley and Sons (1987-1997), Kunkel (1985) Proc. Natl. Acad. Sci. USA 82: 488-92, Kunkel (1988) Method. Enzymol. 85: 2763-6).

In order to introduce mutation into a polynucleotide, a known process such as Kunkel method or Gapped duplex method may be employed using a mutation introducing kit utilizing site-directed mutagenesis such as QuikChange^{™} Site-Directed Mutagenesis Kit (Stratagene), Gene Tailor^{TM} Site-Directed Mutagenesis System (Invitrogen) and TaKaRa Site-Directed Mutagenesis System (Mutan-K, Mutan-Super Express Km, etc.: Takara Bio Inc.).

Furthermore, synoviolin polypeptides comprise fusion proteins added with other peptide sequences. As a peptide sequence (tag) added to the polypeptide of the invention, a sequence that allows easy identification of the protein, a sequence that gives stability upon expressing the protein via a recombination technique, a sequence that allows simple protein purification process or the like may be selected, including influenza virus hemagglutinin (HA), glutathione-S-transferase (GST), polyhistidine tag (6 x His, 10 x His, etc.), maltose-binding protein (MBP).

Since synoviolin is capable of auto-ubiquitination even with its intracytoplasmic domain only, synoviolin used with the present invention may be synoviolin dTM lacking the cell transmembrane domain. For example, MBP-synoviolin dTM-His in which the above tag proteins are fused to the dTM may also be used with the present invention.

### (2) Synoviolin gene or polynucleotide

According to the present invention, synoviolin gene is not particularly limited as long as it is a polynucleotide having the nucleotide sequence represented by SEQ ID NO: 1 or a nucleotide sequence coding for the synoviolin polypeptide of the invention. For example, other than the polynucleotide coding for the amino acid sequence represented by SEQ ID NO: 2, a polynucleotide coding for a mutant polypeptide that has an amino acid sequence having one or more amino acids deleted, inserted, substituted or added in the amino acid sequence represented by SEQ ID NO: 2 and that still maintains the activity as synoviolin may also be used with the present invention.

Herein, the term "polynucleotide" refers to a polymer of bases or base pairs such as deoxyribonucleic acids (DNA) or ribonucleic acids (RNA), including DNA, cDNA, genomic DNA, chemically synthesized DNA and RNA, preferably DNA. The polynucleotide also comprises a polynucleotide including non-natural artificial bases as appropriate.

The synoviolin gene of the invention comprises a nucleotide sequence coding for the amino acid sequence represented by SEQ ID NO: 2. Nucleotide sequences coding for the amino acid sequence comprise the nucleotide sequence represented by SEQ ID NO: 1 as well as nucleotide sequences that differ from the nucleotide sequence represented by SEQ ID NO: 1 due to degeneracy of genetic codes. When the polynucleotide of the invention is used for expressing a polypeptide by genetic engineering process, a nucleotide sequence having high expression efficiency can be selected and designed considering codon usage frequency of the used cell (Grantham et al. (1981) Nucleic Acids Res. 9: 43-74). The polynucleotide coding for synoviolin may be one that includes modified nucleotides.

Moreover, the synoviolin gene of the invention includes a polynucleotide coding for a polypeptide that hybridizes with a polynucleotide having the nucleotide sequence represented by SEQ ID NO: 1 or a sequence complementary to that nucleic sequence under stringent conditions and that still maintains the activity as synoviolin. Such a synoviolin gene may be obtained from a cDNA library or a genome library by a known hybridization method such as colony hybridization, plaque hybridization, and southern blotting using the polynucleotide having the nucleotide sequence represented by SEQ ID NO: 1 or a fragment thereof as a probe. Preparation of a cDNA library can be reviewed in "Molecular Cloning, A Laboratory Manual 2nd ed." (Cold Spring Harbor Press (1989)). Alternatively, a commercially available cDNA library or genome library may be used.

As to hybridization conditions according to the present invention, stringent conditions include, for example, "2 x SSC, 0.1% SDS, 25°C", "1 x SSC, 0.1% SDS, 25°C" and "0.2 x SSC, 0.1% SDS, 42°C "; more stringent conditions include, for example, "0.1 x SSC, 0.1% SDS, 68°C ". Those skilled in the art can determine conditions for obtaining a polynucleotide coding for the synoviolin gene of the invention considering other conditions such as salt concentrations of buffers, temperature, probe concentration, probe length, reaction period or the like.

Detailed procedure of hybridization method can be reviewed in "Molecular Cloning, A Laboratory Manual 2nd ed." (Cold Spring Harbor Press (1989), specifically Section 9.47-9.58).

The polynucleotides of the invention include a polynucleotide coding for an amino acid sequence having one or more amino acids deleted, inserted, substituted or added in the amino acid sequence represented by SEQ ID NO: 2, or a sequence complementary to this polynucleotide sequence. These polynucleotides may be prepared according to a known method such as the site-directed mutagenesis mentioned above. Mutagenesis may also be carried out by using a commercially available kit described above.

The nucleotide sequence of the polynucleotide of the invention may be confirmed by conventional sequencing. For example, sequencing may be carried out by dideoxynucleotide chain termination method (Sanger et al. (1977) Proc. Natl. Acad. Sci. USA 74: 5463) or the like. Alternatively, an appropriate DNA sequencer (e.g., ABI PRISM (Applied Biosystems)) may be employed to analyze the sequence.

Alternatively, according to the present invention, a gene or a polynucleotide coding for synoviolin may be obtained from the synovial cells from an RA patient described above by cloning according to a known method (Nucleic Acid Res. 16:7583-7600, 1988). For example, a gene coding for synoviolin may be obtained through amplification by PCR using a cDNA library from synovial cell or the like as a temperate. Specifically, a cDNA library is obtained based on mRNA extracted from synovial cells from an arthritis-developing tissue of an RA patient, which are collected as synovial tissue or cultured cells (Nucleic Acid Research, 16, 7583, 1988). Next, using a probe designed based on the nucleic sequence represented by SEQ ID NO: 1 in WO 02/052007 publication, a clone hybridized from this library is screened to isolate a gene coding for synoviolin.

### (3) Synoviolin expression vector and host

Although an example of a preferable host/vector system for integrating a gene or a polynucleotide coding for synoviolin described in (ii) above into an appropriate expression system includes an *E*. *coli*/plasmid system, the present invention is not particularly limited thereto. For example, an expression vector pMAL can express a foreign gene in *E. coli.* as a fusion protein with a maltose-binding protein (MBP) (Gene, 67:31-40, 1988). A plasmid integrated with a gene coding for synoviolin is transformed into an *E*. *coli* strain such as BL21 protease-deficient strain by heat shock. After an appropriate period of culture, isopropylthio-(β-D-galactosidase (IPTG) is added to induce an expression of MBP-fused synoviolin (MBP-synoviolin). Since this MBP acts as a tag, an expression product, namely MBP-synoviolin, may readily be separated and purified with an amylose bead column.

Other than those mentioned above, the followings may also be applied to a host/vector system for obtaining a synoviolin recombinant. First, in the case where a bacterium is used as a host, expression vectors for fusion proteins utilizing various tag proteins such as histidine tag, HA, FLAG and GST are commercially available. Among yeasts, *Pichia* yeast is known to be effective for expressing a protein with a sugar chain. In terms of adding a sugar chain, an expression system utilizing a baculovirus vector, which uses an insect cells as a host is also useful (Bio/Technology, 6:47-55, 1988). Furthermore, a mammal cell has been utilized to carry out transfection of a vector using a promoter such as CMV, RSV, EF-1α or SV40, and any of these host/vector systems may be used as an expression system of synoviolin. Virus vectors such as a retrovirus vector, an adenovirus vector and an adeno-associated virus vector can be used to introduce a gene coding for synoviolin.

Synoviolin resulting as an expression product of the host/vector system mentioned above may be used as follows. For example, a vector expressing tagged synoviolin is transfected into a cell. Then, cells expressing synoviolin are fractured, added with [³²P]-labeled ubiquitin for reaction, and immunoprecipitated with an anti-tag antibody. The precipitate is separated by SDS-PAGE and ubiquitin ligase activity of synoviolin is detected by autoradiography (Hashizume, R. et al., J. Biol. Chem. 276, 14537-14540, 2001).

### 3. Solubilizing and reacting MBP-synoviolin-His fusion protein with plate

Hereinafter, MBP-synoviolin-His, a fusion protein of synoviolin, MBP and His, will be described as an example.

A known method such as centrifuging a culture solution containing the transformed cells is used to collect the cells or the cultured strain. Strain pellets suspended in an appropriate buffer are subjected to a treatment such as sonication, lysozyme and/or freeze-thaw in ice to disrupt the strain or the cells to elute MBP, His-fused synoviolin. In order to promote this solubilization, a surfactant such as TritonX-100 and Tween 20 may be used. Usually, the fusion protein as the eluted expression product may be purified by chromatography using, for example, amylose beads or nickel agarose beads. According to purpose, the tag portion, MBP or His, may be separated from the fusion protein with protease so that the protein of interest can be used as a unit in an assay.

MBP-synoviolin-His is likely to form an assembly after being eluted from the amylose beads or nickel agarose beads used for purification and being dialyzed. Thus, the solubilized bacterial cells containing the synoviolin-fusion protein is reacted with a solid support such as a plate without separation/purification to obtain a complex of the support and the MBP-synoviolin-His fusion protein for subsequent use. This method is much simpler than using purified synoviolin and is preferable in terms of preventing assembly formulation. This assembly is caused by the fact that the MBP-synoviolin-His fusion protein has a auto-ubiquitination activity exhibited by synoviolin and still maintains the activity even after immobilization on a solid support.

As the solid support, an immunoplate (multiwell plate) may be used, including, for example, a 96-well plate and a 384-well plate. When the solubilized bacterial cells containing the synoviolin fusion protein are directly reacted with the plate, proteins other than the synoviolin-fusion protein of interest are also immobilized on the plate. Therefore, in order to bind only the MBP-synoviolin-His fusion protein of interest to the plate, the plate is preferably immobilized with anti-MBP antibody beforehand. Since a plate immobilized with an anti-MBP antibody specifically binds a protein with an MBP-tag, when the solubilized cells are reacted with the plate, the MBP-synoviolin-His fusion protein of interest is selectively bound to the plate. The antibody immobilized to the plate may be any antibody that binds to a tag bound to synoviolin as a fusion protein. For example, when a fusion protein of synoviolin and GST is used, the antibody immobilized to the plate may be either a monoclonal or polyclonal antibody as long as it is an anti-GST antibody.

Immobilization of anti-MBP antibody to a plate in the case of a 96-well plate may be carried out by incubating 1 µg/well of anti-MBP antibody in a buffer such as PBS or a citric acid buffer at 4°C overnight.

With this anti-MBP antibody immobilized plate, the MBP-synoviolin-His fusion protein produced can directly be bound to the plate without extraction or purification from the bacterial cell, and unbound substances can readily be removed or separated by washing the plate after the auto-ubiquitination reaction described below. The solubilized bacterial cells including the fusion protein described above are used in an amount of 0.3-7 µl, preferably 0.5-3 µl per well of the 96-well plate for reaction.

### 4. Ubiquitin

According to an ELISA method (described below) used with the present invention, ubiquitin is bound to synoviolin of the MBP-synoviolin-His fusion protein bound to a plate via ubiquitin ligase activity of the synoviolin itself.

The type of ubiquitin used with the invention is not limited as long as it binds to synoviolin via ubiquitin ligase activity of the synoviolin. A polypeptide having the amino acid sequence represented by SEQ ID NO: 4 may be used as the ubiquitin of the present invention. The nucleotide sequence of DNA coding for the polypeptide represented by SEQ ID No: 4 is represented by SEQ ID NO: 3. The ubiquitin polypeptide used with the present invention may be one of the variants for SEQ ID NO: 4 described for synoviolin polypeptide in (i). Moreover, the ubiquitin gene or the polynucleotide used with the present invention may be one of the variants described for synoviolin gene or polynucleotide in (ii).

The nucleotide sequence coding for ubiquitin and the amino acid sequence of ubiquitin are represented by SEQ ID NOS: 3 and 4, respectively.

For example, when ubiquitin (Ub) is produced from a host by a genetic engineering technique, GST-HA-Ub added with GST and HA tags may be used. When a tag or the like is added, an activity of ubiquitin to bind to synoviolin by a ubiquitin ligase activity of synoviolin needs to be maintained in the ELISA method of the invention. The GST-Ha-Ub described above may be obtained by expressing it using an expression vector/host as described in (iii). For example, Ub gene is inserted into a vector (pGEX) for the GST and HA-tagged fusion protein to generate a fusion protein in *E*. *coli*. Each fusion protein obtained from solubilized *E*. *coli* supernatant is purified through a glutathione sepharose^{™} 4B (Amersham Biosciences) column to obtain the GST-HA-Ub described above.

In order to purify the fusion protein produced from the host as described above, the following method may be used. Specifically, first, the host that produces the fusion protein is disrupted. The host can be disrupted by employing a method such as sonication, lysis with enzymes such as lysozyme and French press, alone or in an appropriate combination. Subsequently, an appropriate buffer is added, mixed and subjected to centrifugation to obtain the supernatant. The protein contained in the obtained supernatant is adsorbed using adsorption beads or the like. For example, beads such as glutathione sepharose^{™} 4B (Amersham Biosciences) are used for rotation for an appropriate period of time to allow the produced protein to be adsorbed to the beads. Thereafter, the produced protein may be separated from these beads. As to the separation method, for example, the beads that adsorbed the protein are packed into a column for elution using an appropriate buffer. The eluted protein may be collected after dialysis.

The ubiquitin used with the present invention comprises Pk-His-HA-Ub K48R or BioEase-Ub. Pk-His-HA-Ub K48R is characterized in that polyubiquitination via lysine residue at position 48 is not formed since the lysine residue at position 48 of ubiquitin is substituted with arginine. Pk-His-HA-Ub K48R is a protein obtained by binding HA tag, His-tag and Pk to a variant obtained by mutating lysine at position 48 of Ub into arginine. By using Pk-His-HA-Ub K48R in the method of the present invention, the background level is reduced, enabling an assay at a high S/N ratio. BioEase-Ub is a protein obtained by inserting ubiquitin gene into pET104-PEST^{™} (Invitrogen) to produce a fusion protein in *E. coli,* upon which biotin is covalently added to a specific lysine residue in BioEase^{™}Tag (biotinylated *in vivo*) and purifying the fusion protein obtained from the solubilized supernatant with beads such as Streptavidin Agarose (Invitrogen).

By using BioEase-Ub in the method of the present invention, the background level is significantly reduced, enabling an assay at a very high S/N ratio.

According to the present invention, ubiquitin or biotinylated ubiquitin is preferably used in an amount of 1.7 µg/well in a 96-well plate.

The ubiquitin of the invention may also be labeled before use. A label for ubiquitin may be any label that is detectable with an appropriate chemical or physical detecting means. For example, fluorescent materials such as fluorescamine and fluorescein isothiocyanate, enzymes such as peroxidase, alkaline phosphatase and malate dehydrogenase, and luminescent materials such as luciferin and luminal may be used to label ubiquitin. Also, the ubiquitin of the invention may be biotinylated before use. A biotinylation method may be performed *in vitro* or *in vivo.* An example of an *in vitro* biotinylation method includes incubation with NHS (N-hydroxysuccinimide)-biotin (BIO RAD) at room temperature for 2 hours. An example of *in vivo* biotinylation method comprises transforming ubiquitin cloned into a vector such as pET104-PEST and producing a biotinylated protein in the resulting transformant. The labeled ubiquitin is analyzed by means capable of detecting each label, by which ubiquitin is quantitated. In the case of using the above-described biotin label, the label may be detected using avidin-alkaline phosphatase.

When MBP-synoviolin-His exemplified in Section 3 above is used in an experiment, MBP-His lacking synoviolin may be used as a control of auto-ubiquitination reaction.

### 5. Ubiquitination of synoviolin

Ubiquitination is a process in which ubiquitin-activating enzyme (E1), ubiquitin-conjugating enzyme (E2) and ubiquitin ligase (E3) cooperate to successively bind ubiquitin to a substrate protein. As described above, auto-ubiquitination of synoviolin means that synoviolin having a ubiquitin ligase activity becomes the substrate protein for ubiquitination by itself and binds ubiquitin by itself without depending on other ubiquitin ligase.

Enzymes such as ubiquitin-activating enzyme (E1) and ubiquitin-conjugating enzyme (E2) are commercially available and may be used appropriately. E1 is used in a range of 10-50 ng, preferably 20-40 ng and E2 is used in a range of 0.1-0.5 µg, preferably 0.2-0.3 µg.

As a low molecular weight reactive factor required for ubiquitination, Mg salts such as MgCl₂ and MgSO₄, ATP, NAF, DTT (dithiothreitol), okadaic acid or the like may appropriately be selected for use. These compounds are also commercially and readily available.

Moreover, a buffer for dissolving the enzymes or reagents, a washing buffer, a buffer used for determination, a buffer used to terminate the reaction and the like may be any known buffer (e.g., Tris-HCI) as long as it does not deactivate the enzymes or interfere with the reactions.

The auto-ubiquitination reaction of synoviolin may be carried out under the following conditions. To a given amount of buffer (pH 6-8) as a reaction solvent, the above-mentioned predetermined amounts of reaction substances (other than synoviolin or its active derivative) necessary for auto-ubiquitination of synoviolin are added and incubated at room temperature for 5-30 minutes. Then, synoviolin, its active derivative or an active derivative of immobilized synoviolin is added to initiate auto-ubiquitination reaction of synoviolin. Incubation is carried out at a constant temperature in a range of room temperature to 37°C for a predetermined period in a range of 20-120 minutes, and a predetermined amount of a terminating buffer (containing 0.2 M boric acid buffer, TritonX-100 and EDTA) is added to terminate the reaction. The amounts of ubiquitin binding and not binding to synoviolin are quantified to determine the degree of auto-ubiquitination. To this end, a labeled ubiquitin or an anti-ubiquitin antibody is preferably used.

### 6. Assay kit

The present invention not only relates to the above-described assay method but also relates to a combination of means for the assay. A kit according to a specific embodiment of such combination comprises at least:
(i) synoviolin or an active derivative thereof, ubiquitin, ubiquitin-activating enzyme (E1), ubiquitin-conjugating enzyme (E2), a low molecular weight reactive factor required for ubiquitination and buffers used for ubiquitination of synoviolin; and
(ii) reagents used in the step of determining the amounts of ubiquitin binding or not binding to synoviolin in the reaction solution resulting from the above reaction (e.g., washing solution used for ELISA, a ubiquitin labeling enzyme, o-phenylenediamine, sulfuric acid, etc.).

The multiwell plate for reaction may be, for example, a 96- or 384-well plate. Those skilled in the art will readily select the type of the plate. If the kit includes a multiwell plate, the plate may be immobilized with anti-MBP antibody beforehand.

Any of those mentioned in the description of assay above may be used favorably as synoviolin, ubiquitin, necessary enzymes, a low molecular weight reactive factor required for ubiquitination and reagents used in the step of determining the amount of ubiquitin.

Apart from the "reaction buffer", the "buffers" included in the kit may comprise a "washing solution". Examples of the "washing solution" include the same solvent as the "reaction buffer", other aqueous solvents, phosphate buffers and Tris-hydrochloric acid buffer. Both "washing solution" and "reaction buffer" may be buffers of the same type, and the washing and eluting can be fulfilled by altering the liquid property of the buffer, specifically by changing the ion strength, pH and types of salts contained.

Each of these components included in the kit may be prepared separately or coexist as far as circumstances allow. The kit may include solutions or may include condensed forms of components that can be blended into such solutions.

If necessary, the kit may further include auxiliary agents, a purpose-designed container, other necessary accessories, instruction and the like.

The kit of the invention is based on ELISA method. ELISA method (enzyme-linked immunosorbent assay) is an assay in which a substance that binds to an enzyme-labeled antigen (or an antibody) (usually labeled with an enzyme) or an amount thereof is detected using an enzyme activity. The kit of the invention is based on auto-ubiquitination reaction of synoviolin rather than the antigen-antibody reaction described above. Specifically, the kit of the invention utilizes an enzyme activity to detect ubiquitin binding to synoviolin or an amount thereof by using enzyme-labeled ubiquitin. According to the present invention, the assay based on auto-ubiquitination reaction may also be referred to as an ELISA method.

### 7. Screening substance having activity of regulating ubiquitination

A method of the invention for screening a substance having an activity of regulating ubiquitination of synoviolin is based on the procedure below.

The method uses synoviolin or an active derivative thereof and comprises at least the steps of:
(i) contacting ubiquitin with synoviolin or an active derivative thereof in a well of a multiwell plate in the presence or in the absence of a candidate substance;
(ii) determining a ubiquitination activity of synoviolin or the active derivative thereof by carrying out the above-described assay procedure in the well;
(iii) comparing the results obtained from (i) and (ii) above with a result from a simultaneously-obtained control to detect an activity of the candidate substance to regulate auto-ubiquitination activity of synoviolin; and
(iv) selecting the candidate substance with a high regulating activity.

In this case, the control is preferably obtained by carrying out the steps (i) and (ii) under the conditions where the candidate substance is absent. Alternatively, a control with a lower concentration of the candidate substance may also be used. For example, instead of the candidate substance, a molecule known to have an activity of promoting or inhibiting the auto-ubiquitination of synoviolin may be used in the steps (i) and (ii), and substances with higher regulating activities than that molecule may be selected.

In screening such a substance, the activity of the candidate substance to regulate a ubiquitination activity of synoviolin is distinguished as follows. If the amount of ubiquitin binding to synoviolin is increased due to the presence of the candidate substance, this substance is determined to promote ubiquitination of synoviolin. If the amount of ubiquitin binding to synoviolin decreases due to the presence of the candidate substance, this substance is determined to inhibit ubiquitination of synoviolin. For example, a substance that inhibits an interaction between synoviolin and a ubiquitin-activating enzyme and/or a ubiquitin-conjugating enzyme can be determined to effectively inhibit ubiquitination of synoviolin.

When a compound that influences ubiquitination of synoviolin is subjected to screening, a large size low molecular weight compound library needs to be screened in a high-throughput manner. Therefore, a highly sensitive assay that requires a short period of time is desirable to be employed. Thus, according to the present invention, similar to the assay method described above, a multiwell plate that allows simultaneous treatments of multiple samples is used and synoviolin or an active derivative thereof is immobilized on a solid support for reaction so that only the reaction product is remained in the well of the plate to determine the amount of bound ubiquitin directly on the multiwell plate.

Specifically, in order to realize the high-throughput screening method described above, an assay method is preferable in which a complex obtained by directly reacting a solubilized bacterial cell containing synoviolin-His fusion protein described above as the synoviolin or an active derivative thereof with an anti-MBP antibody-immobilized plate.

For detection, since absorbance determination is continually carried out on the plate used for the reaction above, efficiency of quantitating ubiquitin is markedly promoted. As the absorbance determination system for the reaction product, a conventionally used microtiter plate that treats multiple samples in a short time may be used. When a high-density plate of 384 wells or more is used, a device that allows imaging detection based on a CCD camera may be also be used.

According to the screening method of the invention, the screened substance, a low molecular compound, a salt or a derivative thereof that was found to inhibit ubiquitination of synoviolin is comprised in the scope of the invention.

According to the above-described method, a substance having an activity of regulating ubiquitination of synoviolin can be selected. The high-throughput screening method described above may be applied to a search for a compound that influences ubiquitination of other protein that plays a role in signal transduction involving synoviolin.

Hereinafter, the present invention will be described in more detail by way of examples. The present invention, however, is not limited to these examples.

Hereinafter, unless otherwise stated, "%" refers to "% by weight"

### EXAMPLE 1

This example aimed at preparing biotinylated GST-HA-Ub used for detecting *in vitro* auto-ubiquitination of synoviolin by ELISA.

First, GST-HA-Ub was prepared by inserting cDNA containing the entire region of ubiquitin into a vector (pGEX) for a GST and HA fusion protein, producing the fusion protein in *E.coli,* and purifying with a glutathione sepharose^{™} 4B (Amersham Biosciences) column.

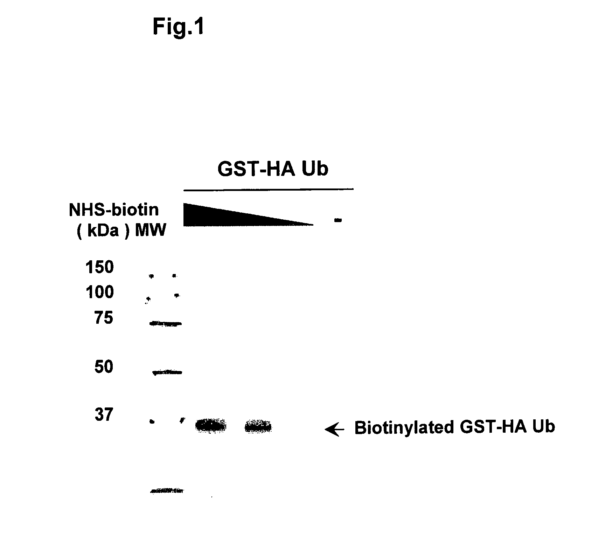

Next, GST-HA-Ub obtained above was labeled with NHS (N-hydroxysuccinimide)-biotin for biotinylation. This biotinylated GST-HA-Ub was subjected to SDS-PAGE, transferred to a nitrocellulose membrane and detected with a biotin/avidin-alkaline phosphatase system. As a result, GST-HA-Ub was biotinylated concentration-dependently of NHS-biotin used for reaction (Figure 1).

### EXAMPLE 2

This example aimed at confirming *in vitro* auto-ubiquitination of synoviolin using western blotting.

### (1) In vitro auto-ubiquitination reaction

The following composition was prepared in a tube to the indicated final concentrations and allowed to react in a thermostatic bath at 37°C for 2 hours: 50 mM Tris-HC1 (pH 7.5), 5 mM MgCl₂, 2 mM ATP, 0.267 µg E1, 0.333 µg E2, 1.7 µg biotinylated GST-HA-Ub, 1 µg MBP-synoviolin dTM Hisₓ₁₂.

E1 (derived from yeast) used for the reaction was purchased from Affiniti. E2 was obtained by inserting UbcH5c into a vector (pET) for a His-tagged fusion protein, producing the fusion protein in *E*. *coli* and purifying with an Ni-NTA column (Qiagen). The biotinylated GST-HA-Ub was one prepared in Example 1. MBP synoviolin ΔTM-Hisₓ₁₂ was obtained by fusing synoviolin lacking the cell transmembrane domain (TM) (Synoviolin ΔTM) with MBP. MBP synoviolin dTM-Hisₓ₁₂ was obtained by producing the fusion protein in *E*. *coli* and purifying with an Ni-NTA column (Qiagen).

### (2) Detection of auto-ubiquitination by western blotting

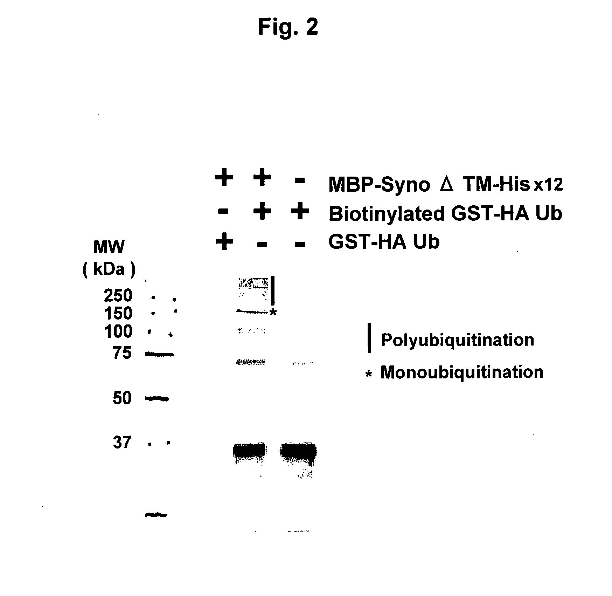

Following *in vitro* auto-ubiquitination reaction in (1), the sample was subjected to SDS-PAGE and transferred to a nitrocellulose membrane. Next, the nitrocellulose membrane was blocked with 5% skimmed milk, added with 1000-fold diluted avidin-alkaline phosphatase and shaken for 30 minutes. Then, the nitrocellulose membrane was washed with TBS-T and detected with Alkaline phosphatase conjugate substrate kit (BIO RAD). The results are shown in Figure 2. As can be appreciated from Figure 2, a band of synoviolin ubiquitinated with biotinylated GST-HA-Ub was detected.

### EXAMPLE 3

This example aimed at constructing an ELISA-method-based system for detecting *in vitro* auto-ubiquitination of synoviolin using biotinylated GST-HA-Ub.

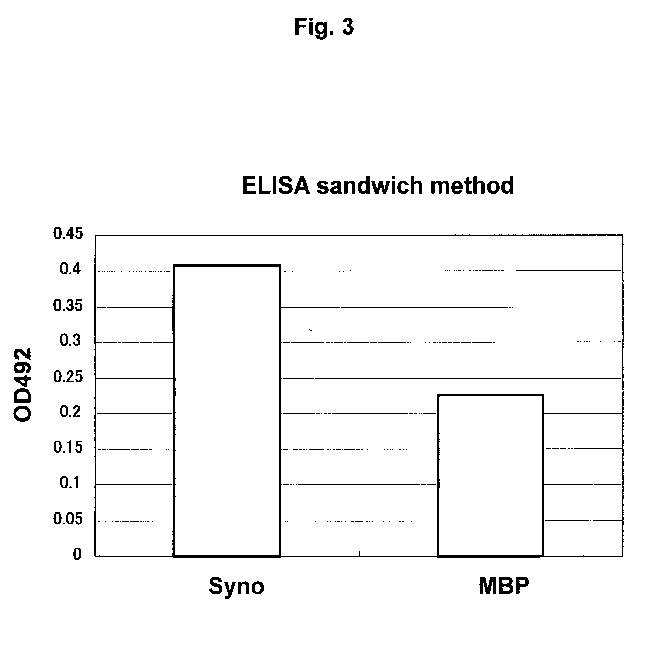

According to this method, 1 µg/well of anti-MBP antibody was coated onto a 96-well plate at 4°C overnight, blocked with 1% BSA at room temperature for 4 hours, washed with 0.01 % Tween 20/PBS(-) and reacted with MBP-synoviolin ΔTM Hisₓ₁₂ or MBP-Hisₓ₆ in the well at 4°C overnight. Thereafter, *in vitro* auto-ubiquitination reaction using biotinylated GST-HA-Ub was carried out according to the method described in Example 2 above. After 2 hours of reaction at 37°C, the plate was washed with 0.01% Tween 20/PBS(-), blocked with 1% BSA at room temperature for an hour, washed with 0.01% Tween 20/PBS(-), added with 1000-fold diluted avidin-alkaline phosphatase and allowed to stand still for 30 minutes. After washing with 0.01% Tween 20/PBS, Alkaline phosphatase substrate kit (BIO RAD) was added and left to stand still for 5 minutes, and the reaction was terminated by adding 0.4N sodium hydroxide. Thereafter, absorbance at 420 nm was determined. As a result, auto-ubiquitination of a larger amount of MBP-synoviolin ΔTM Hisₓ₁₂ (Figure 3, "Syno") was found by ELISA method compared to MBP-Hisₓ₆ (Figure 3, "MBP") (Figure 3).

### EXAMPLE 4

In this example, to reduce the background in detecting the auto-ubiquitination activity of in vitro synoviolin auto-ubiquitination assay using western blotting method (WB), the background levels were compared between GST-HA-Ub and Pk-His-HA-Ub K48R in the reaction.

Pk-His-HA-Ub K48R is a protein in which an HA tag, a His-tag and a Pk site are bound to a variant obtained by mutating lysine at position 48 of Ub into arginine. Pk-His-HA-Ub K48R was prepared by inserting His-HA-ubiquitin (K48R) gene with Pk site into pET vector, producing the fusion protein in *E*. *coli* and purifying with a Ni-NTA column (QIAGEN).

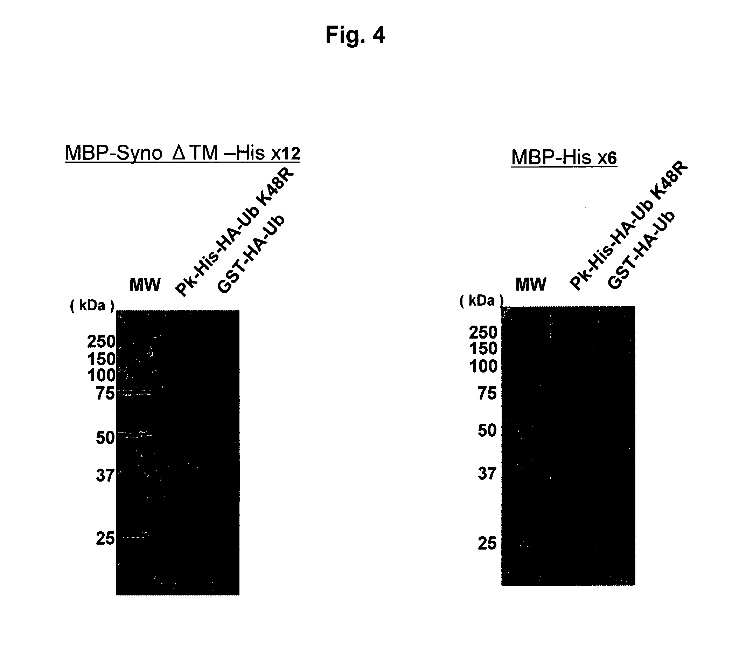

In this method, GST-HA-Ub or Pk-His-HA-Ub K48R was used in a usual *in vitro* auto-ubiquitination reaction described in Example 2. Following reaction at 37°C for 2 hours, the reaction solution was subjected to SDS-PAGE and transferred to a nitrocellulose membrane. The nitrocellulose membrane was blocked with 5% skimmed milk, added with 2500-fold diluted (including 0.5% skimmed milk) α-HA (3F10) (Roche), shaken for an hour and washed with TBS-T. Next, α-rat Ig HRP was added, shaken, washed with TBS-T and detected with ECL prepared in the laboratory. As a result, the use of Pk-His-HA-Ub K48R was proved to reduce the background level (Figure 4).

### EXAMPLE 5

This example aimed at examining whether Pk-His-HA-Ub K48R, which was found effective for detecting *in vitro* auto-ubiquitination by western blotting method in Example 4, was also effective for detection by ELISA method.

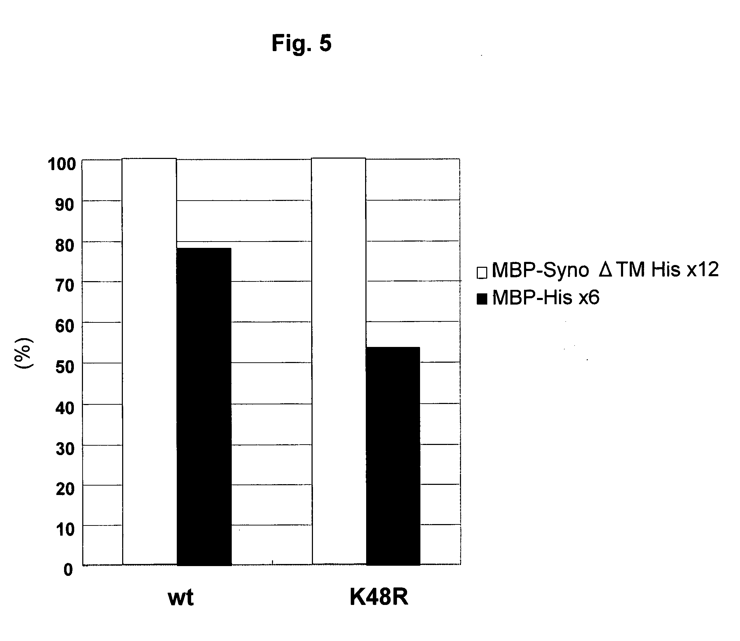

In this method, 1 µg/well of anti-MBP antibody was coated onto a 96-well plate at 4°C overnight, blocked with 1% BSA at room temperature for 4 hours, washed with 0.01 % Tween 20/PBS(-) and reacted with MBP-synoviolin ΔTM Hisₓ₁₂ or MBP-Hisₓ₆ in the well at 4°C overnight. Thereafter, *in vitro* auto-ubiquitination reaction using GST-HA-Ub (Figure 5, "wt") or Pk-His-HA-Ub K48R (Figure 5, "K48R") was carried out. After 2 hours of reaction at 37°C, the plate was washed with 0.01 % Tween 20/PBS(-). Then, the plate was blocked with Block Ace at room temperature for an hour, washed with 0.01 % Tween 20/PBS(-), added with 2500-fold diluted (including 1/10 Block Ace) α-HA (3F10) (Roche) and allowed to stand still for 60 minutes. After washing the plate with 0.01 % Tween 20/PBS(-), α-rat IgG HRP was added and left to stand still for 30 minutes. After washing with 0.01% Tween 20/PBS(-), O-phenylenediamine was added in 100 µl/well, left to stand still for 15 minutes and added with 2N sulfuric acid to terminate the reaction. Absorbance at 492 nm was determined.

As a result, similar to the results from WB in Example 3, use of Pk-His-HA-Ub reduced the background level while significant difference was confirmed between MBP-synoviolin ΔTM Hisₓₗ₂ and MBP-Hisₓ6 (Figure 5).

### EXAMPLE 6

This example aimed at constructing a system for detecting *in vitro* auto-ubiquitination of synoviolin using biotinylated ubiquitin by ELISA.

### (1) Cloning

Ubiquitin or each of KR variants (5 types) was cloned into pET104.1-DEST using Gateway system (Invitrogen) (pET104.1-DEST/Ub).

### (2) Purification of in vivo biotinylated protein

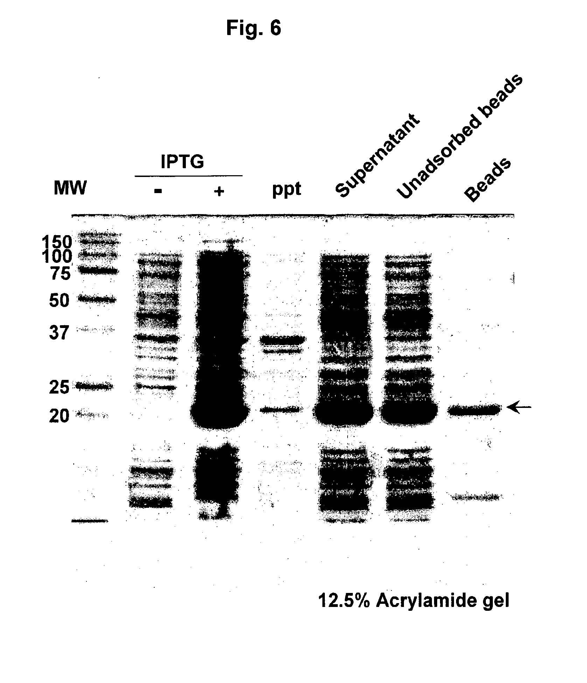

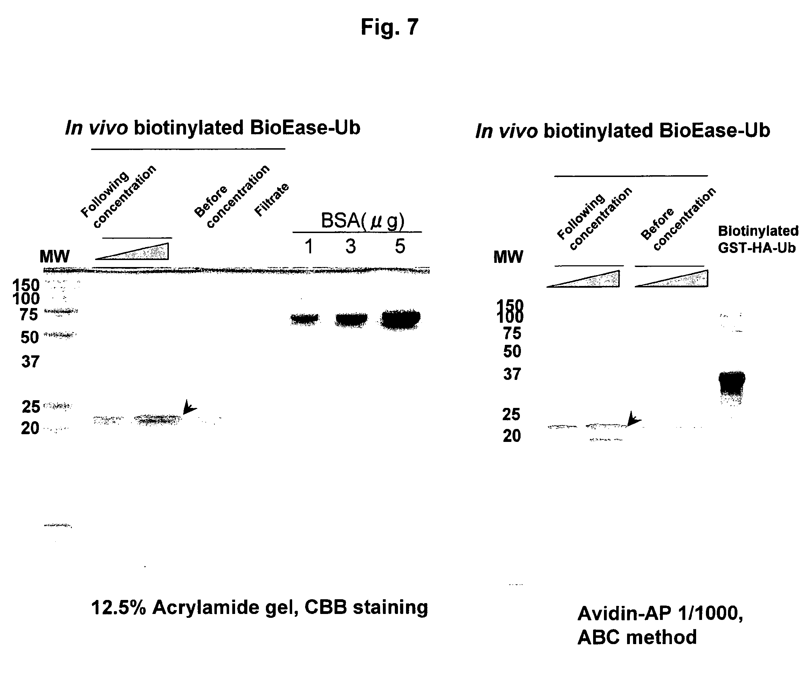

BL21 Star (DE3) was transformed with pET104.1-DEST/Ub, added to 10 ml ofL-broth containing ampicillin and shake cultured at 37°C overnight. Ten ml of this pre-culture was added to 500 ml of L-broth containing ampicillin and shake cultured at 37°C. Absorbance at 600 nm was determined. Once absorbance was confirmed to exceed 0.4, IPTG was added to a final concentration of 1 mM and shake cultured at 37°C for 4 hours. After harvesting, the strain was suspended in PBS(-), added with lysozyme, allowed to stand still in ice for 10 minutes and sonicated. A washing buffer was added to the following final concentrations: 1% Triton-X, 1 mM EDTA, 1 mM PMSF, 0.5M NaC1, 5 mM 2-ME and 1 µg/ml aprotinin. The resultant was passed through a 21 G syringe twice and centrifuged at 7,000 rpm at 4°C for 30 minutes to obtain supernatant. Streptavidin Agarose (Invitrogen) was used as beads. Streptavidin Agarose was washed for three times with 5 bed vol. of washing buffer. The washed beads were added to the supernatant and rotated at 4°C overnight. The beads were washed for three times with 5 bed vol. of the washing buffer, suspended in 1 bed vol. of 1 x PBS(-) and packed into a column. The protein was eluted with 0.5 bed vol. of eluting buffer (5 mM biotin, 1 x PBS) for 10 times. The eluted protein was dialyzed with 1 x PBS(-) overnight and collected. Then, the protein was concentrated with Centricin YM-10 (MILLIPORE). As a result, *in vivo* biotinylated BioEase-Ub was obtained (Figures 6 and 7).

### (3) Confirmation of in vitro auto-ubiquitination of synoviolin using western blotting (WB) method

In a general *in vitro* auto-ubiquitination reaction, *in vivo* biotinylated BioEase-Ub, *in vitro* biotinylated Pk-His-HA-Ub WT or *in vitro* biotinylated GST-HA-Ub was used. The followings were placed in a tube to the indicated final concentrations and reacted in a thermostat bath at 37°C for 2 hours: 50 mM Tris-HCl (pH 7.5), 5 mM MgCl₂, 2 mM ATP, 0.125 µg E1, 0.166 µg E2, 0.85 µg Ub, 0.5 µg MBP-synoviolin dTM Hisₓ₁₂. Then, the resultant was subjected to SDS-PAGE and transferred to a nitrocellulose membrane. After blocking with 5% skimmed milk, 1000-fold diluted avidin-alkaline phosphatase was added and shaken for 30 minutes. Thereafter, the resultant was washed with TBS-T and detected with Alkaline phosphatase conjugate substrate kit (BIO RAD). *In vitro* biotinylated Pk-His-HA-Ub WT, *in vivo* biotinylated BioEase-Ub and *in vitro* biotinylated GST-HA-Ub were used to perform *in vitro* auto-ubiquitination.

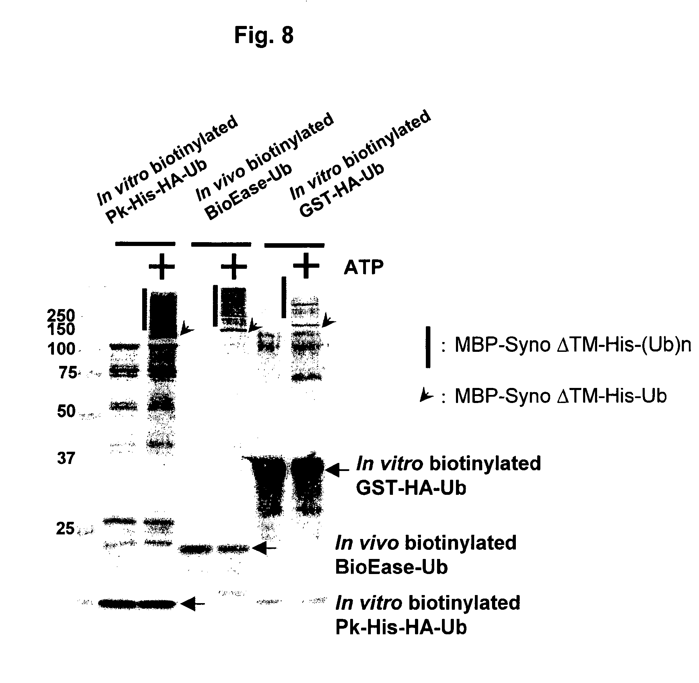

As a result, monoubiquitination and polyubiquitination of synoviolin were confirmed in *in vitro* biotinylated Pk-His-HA-Ub WT, *in vivo* biotinylated BioEase-Ub and *in vitro* biotinylated GST-HA-Ub (Figure 8). Reduction in the background level was particularly significant in *in vivo* biotinylated BioEase-Ub.

### EXAMPLE 7

Construction of auto-ubiquitination reaction system of synoviolin by ELISA and assessment of inhibiting activity of compound.

This example aims at constructing a simple system for assaying auto-ubiquitination reaction by ELISA and at evaluating a compound that inhibits auto-ubiquitination reaction.

### (1) Examination of auto-ubiquitination reaction system of synoviolin by ELISA

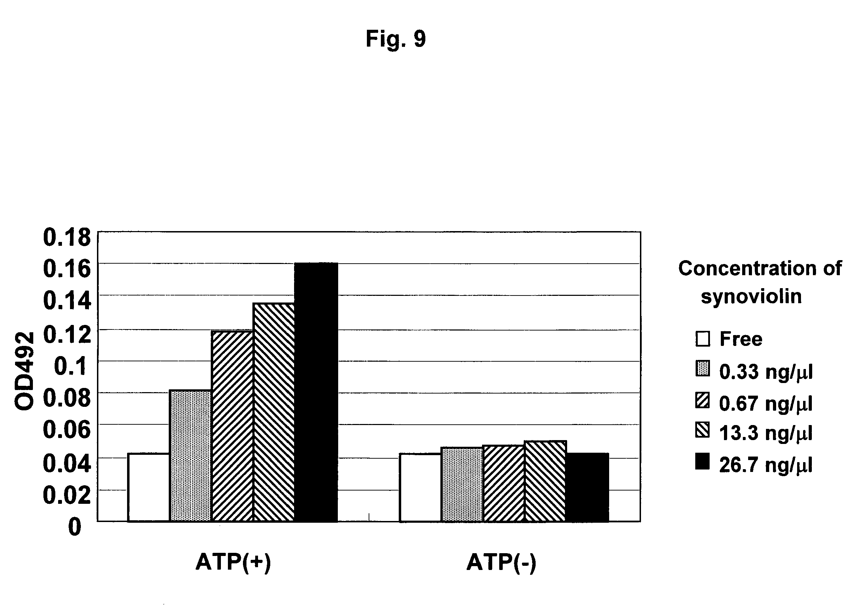

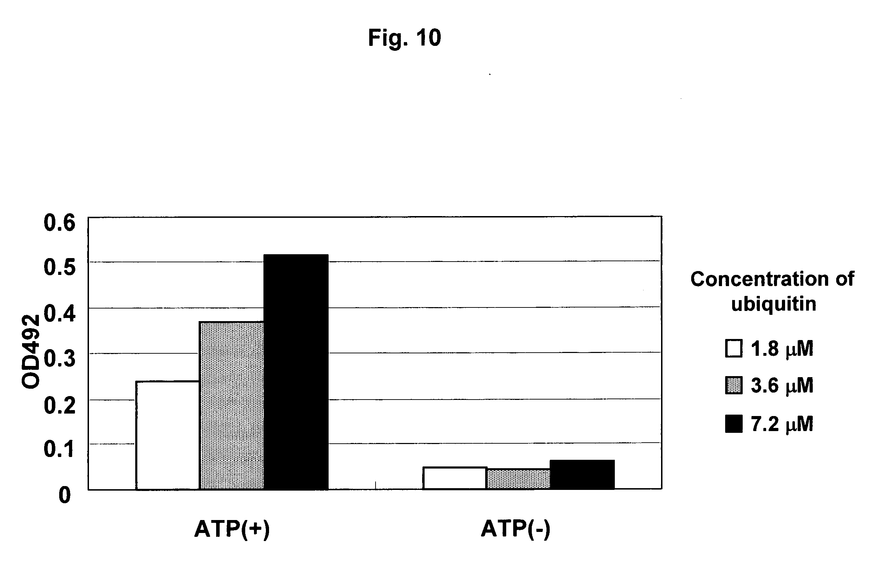

The concentration of MBP-Syno ΔTM-His (Figure 9) or the concentration of PK-His-HA-Ub (Figure 10) was varied to carry out *in vitro* auto-ubiquitination reaction (30 µl) in the presence or absence of ATP. To 30 µl of the reaction solution, 30 µl of 0.5M EDTA was added. MBP-Syno ΔTM-His was bound to an anti-MBP-antibody-coated plate for ELISA, followed by washing and detection with anti-HA antibody.

As a result, in the presence of ATP, an enhancement of the signal was observed concentration-dependently of MBP-Syno ATM-His (Figure 9) or PK-His-HA-Ub (Figure 10).

### (2) Requirement of ATP, PK-His-HA-Ub, E1, E2 and MBP-Syno ΔTM-His

ATP, PK-His-HA-Ub, E1, E2 and MBP-Syno ΔTM-His were combined to carry out *in vitro* auto-ubiquitination reaction (30 µl) and detected according to the same method described above.

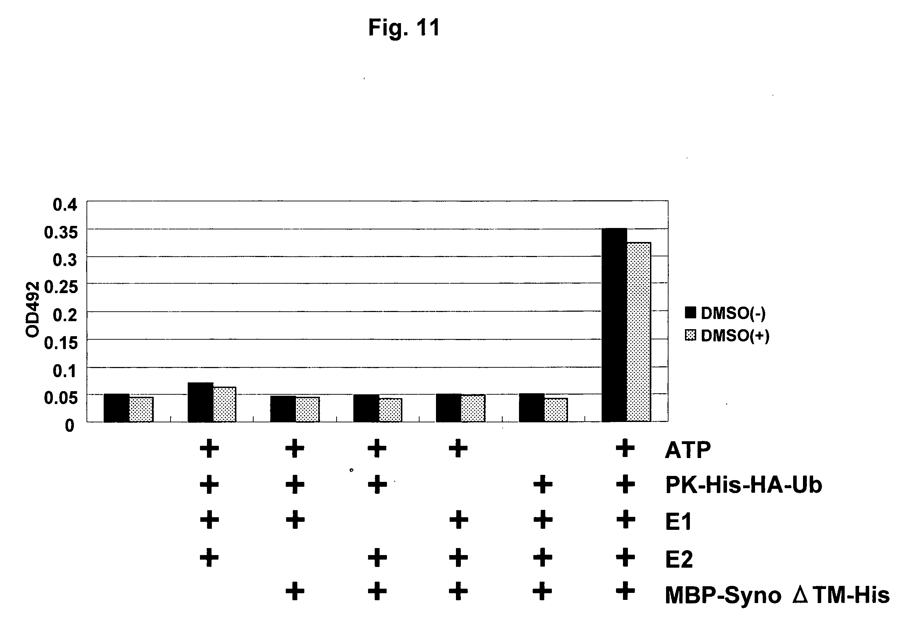

As a result, signal was detected only when all of ATP, PK-His-HA-Ub, E1, E2 and MBP-Syno ΔTM-His were combined (Figure 11).

### (3) Examination of concentrations of primary antibody (rabbit anti-HA antibody) and secondary antibody (anti-rabbit IgG/HRP) upon detection

The dilution ratios of rabbit anti-HA antibody (1/1000, 1/2000, 1/4000) and secondary antibody (1/2500, 1/5000, 1/10000) were varied to carry out *in vitro* auto-ubiquitination reaction (30 µl) in the presence or absence of ATP.

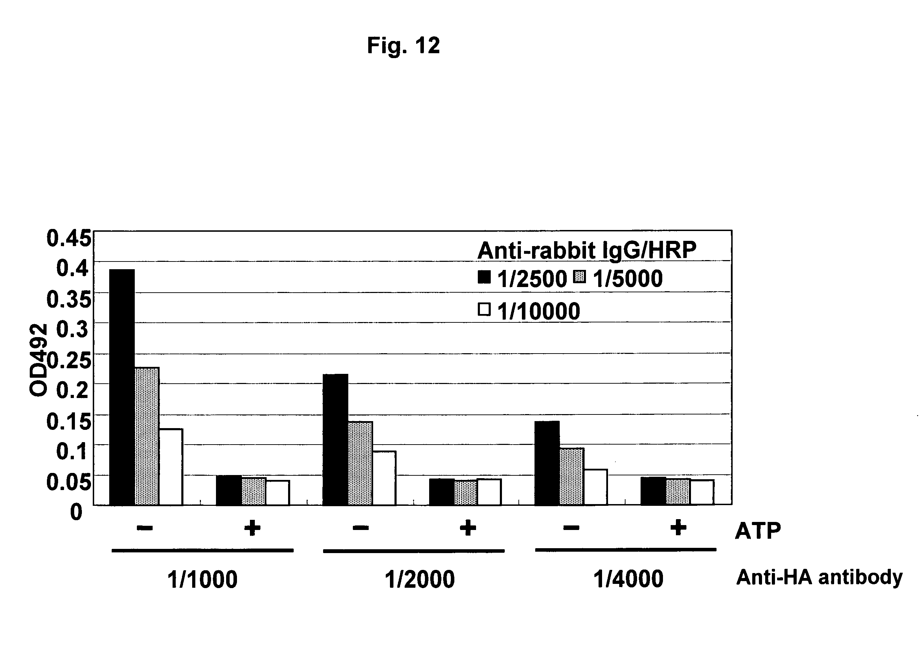

As a result, in the presence of ATP, an enhancement of the signal was observed concentration-dependently of the anti-HA antibody and the secondary antibody (Figure 12).

### INDUSTRIAL APPLICABILITY

According to the assay method of the invention, ubiquitination of synoviolin can be assayed in a highly sensitive manner in a short time. Moreover, since an amount of a sample used can be small, the assay can be carried out at low cost.

According to the screening method of the invention employing such an assay method, a high-throughput screening of a substance that influences ubiquitination of synoviolin can be realized from a very large size low molecular weight compound library.

## Claims

1. An assay method for determining a ubiquitination activity of synoviolin, comprising reacting synoviolin and ubiquitin in a reaction system containing them, and determining an amount of ubiquitin binding to synoviolin in the resulting reaction product.

2. A method according to Claim 1, wherein the step of reacting comprises ubiquitination reaction in a system comprising synoviolin or an active derivative thereof, ubiquitin, a ubiquitin-activating enzyme, a ubiquitin-conjugating enzyme and a low molecular weight reactive factor required for ubiquitination.

3. A method according to either one of Claims 1 and 2, wherein the step of determining comprises determining an amount of ubiquitin binding to synoviolin.

4. A method according to any one of Claims 1 to 3, wherein the active derivative of synoviolin is a fusion protein of synoviolin and a tag protein.

5. A method according to any one of Claims 1 to 4, wherein the step of determining is carried out by reacting biotinylated ubiquitin binding to synoviolin with avidin to quantify the amount of ubiquitin binding to synoviolin.

6. A method for screening a substance having an activity of regulating ubiquitination of synoviolin, comprising the steps of
(i) contacting ubiquitin with synoviolin or an active derivative thereof in the presence or in the absence of a candidate substance;
(ii) determining a ubiquitination activity of the synoviolin or the active derivative thereof; and
(iii) comparing the results obtained from (i) and (ii) above to that from a control to detect an activity of the candidate substance to regulate ubiquitination activity of synoviolin.

7. A kit for ubiquitination assay of synoviolin comprising at least the following for determining ubiquitination of synoviolin:
(i) synoviolin or an active derivative thereof and ubiquitin; and
(ii) reagents used for determining the amount of ubiquitin binding to synoviolin.

8. A kit according to Claim 7, further comprising a ubiquitin-activating enzyme, a ubiquitin-conjugating enzyme, a low molecular weight reactive factor required for ubiquitination and buffers.
